# EUROPÄISCHE PATENTSCHRIFT

(11) **EP 4 346 645 B1**
(45) Veröffentlichungstag und Bekanntmachung des Hinweises auf die Patenterteilung: **24.06.2026**
(21) Anmeldenummer: 23708470.2
(22) Anmeldetag: 28.02.2023
(51) Int. Cl.: A61B 17/16

(54) **DETEKTION VON BOHRERDURCHBRÜCHEN**
DETECTION OF INSTANCES OF DRILL BREAKTHROUGH
DÉTECTION D'INSTANCES DE PERCÉE DE FORET

(30) Priorität: 01.03.2022 DE 102022104782
(43) Veröffentlichungstag der Anmeldung: 10.04.2024
(73) Patentinhaber: Aesculap AG, 78532 Tuttlingen (DE)
(72) Erfinder: RIEDEL, Celine, 07924 Ziegenrück (DE); KAHLER, Thomas-Erwin, 78606 Seitingen-Oberflacht (DE); SILLER, Markus, 88662 Überlingen (DE); AICHER, Philipp, 78532 Tuttlingen (DE); ANDRIS, Robin, 78052 Obereschach (DE)
(74) Vertreter: Winter, Brandl - Partnerschaft mbB
(86) Internationale Anmeldenummer: PCT/EP2023/055040
(87) Internationale Veröffentlichungsnummer: WO 2023/165990

(56) Entgegenhaltungen:
- WO-A1-2016/199152
- WO-A1-2017/078754
- WO-A1-2021/011795
- CN-A- 113 749 726
- US-A1- 2015 066 030

## Beschreibung

### Technisches Gebiet

Die vorliegende Offenbarung betrifft eine Betriebssituations-Erkennungseinrichtung für eine oder einer chirurgische(n) Bohrmaschine, welche bei einem Bohren in einen Knochen einen Durchbruch und / oder eine Durchbruchstendenz als Betriebssituation erkennt und ein Verfahren, welches Betriebszustände/Betriebssituationen insbesondere ein Durchbrechen oder bereits die Tendenz eines Durchbrechens (d.h. das unmittelbare Bevorstehen eines Bohrerdurchbruchs auf der dem Bohrer abgewandten Seite eines Knochens) der chirurgischen Bohrmaschine erkennt.

### Hintergrund der Erfindung

Chirurgische Bohrmaschinen besitzen wichtige Aufgaben im Operationsalltag. Sie dienen beispielsweise zum Vorbohren von Schraublöchern zur Befestigung von Metallplatten bei verletzten oder gebrochenen Knochen in der Orthopädie. Eine weitere Anwendung ist die Trepanation und Kraniotomie (Schädelöffnung) mittels Sicherheitstrepan und Kraniotom in der Neurochirurgie. Eine häufige Operation (OP) ist die Nervenentlastung der Wirbelsäule (Dekompression). Knochenwucherungen werden mit Hilfe des Bohrers entfernt und der Druck auf den Nerv reduziert. Des Weiteren finden chirurgische Bohrmaschinen in der Tumorchirurgie sowohl in Neuro-Wirbelsäulen- und Mund-Kiefer-Gesichts-Chirurgie Anwendung, um tumoröses Gewebe von gesundem Knochen zu resektieren. Eine weitere wichtige Anwendung ist das Vorbohren eines Kanales und Lagers zum Einsetzen eines Cochleaimplantates neben dem Hörkanal.

Solche Operationen sind mit einem erhöhten Risiko für den Patienten verbunden, da hinter dem zu bohrenden Knochen gesundes Gewebe, ggf. Nervenbahnen und Blutgefäße verlaufen, welche leicht verletzt werden können. Eine solche Verletzung kann zu einer erheblichen Schädigung des Patienten führen. Die notwendige / richtige Eindringtiefe des Bohrers ist nicht berechenbar, sondern kann nur geschätzt werden. Studien haben ergeben, dass im Durchschnitt im Kortex 6.33 mm zu tief gebohrt wird. Da Schäden vor allem an der Wirbelsäule und im Gehirn irreversibel sind, hängt die Sicherheit der OP und damit die Sicherheit des Patienten maßgeblich von der Erfahrung des Chirurgen ab.

Die WO 2021 / 011 795 A1 offenbart eine intrakranielle Zugangsvorrichtung mit einer Befestigungsvorrichtung zur Befestigung an einem Schädel eines Patienten. Weiter beinhaltet die Zugangsvorrichtung einen oder mehrere Sensoren, welche vorgesehen und ausgebildet sind, eine Eindringtiefe eines Bohrers in einem Schädelknochen zu bestimmen.

Die WO 2017 / 078 754 A1 offenbart ein Messsystem, das Parameter im Zusammenhang mit einer Bewegung eines medizinischen Fräsers detektieren kann. Beispielsweise kann das Messsystem eine angewandte Kraft und/ oder eine Verschiebung zu einem Referenzpunkt erfassen.

Die CN 113 749 726 A offenbart eine Vorrichtung für Bohrungen in einen Schädelknochen mit einer piezoelektrischen Keramik, welche einen Anpressdruck der Bohrspitze auf den Schädelknochen erfassen kann.

Die US 2015 / 066 030 A1 offenbart eine Vorrichtung zur Bestimmung einer Eindringtiefe eines Instrumentenarbeitsteils in Bezug auf einen Referenzpunkt.

Die WO 2016 / 199 152 A1 offenbart einen Adapter mit einem Sensor, welcher eine plötzliche Verschiebung einer Bohrspitze, beispielsweise bei einem Knochendurchbruch erfassen kann.

### Kurzbeschreibung der Offenbarung

Aufgabe der vorliegenden Offenbarung ist es daher, eine Vorrichtung bereitzustellen, welche zu tiefes Bohren bei einem Bohren in einen Knochen verhindert und Gewebe, was sich hinter dem Knochen befindet, vor einer Penetration oder Verletzung durch den Bohrer schützt und damit die Patientensicherheit erhöht.

Diese Aufgabe wird gelöst durch eine chirurgische Bohrmaschine nach einem der Ansprüche 1 bis 10.

Die Aufgabe wird auch gelöst durch eine Betriebssituations-Erkennungseinrichtung nach Anspruch 11.

Weiterhin ist es Aufgabe der vorliegenden Offenbarung, ein Verfahren bereitzustellen, welches vorbestimmte Betriebszustände einer chirurgischen Bohrmaschine zuverlässig erkennt. Das Verfahren fällt nicht unter den Schutzumfang der Ansprüche.

Konkret wird die Aufgabe, zu tiefes Bohren in den Knochen zu verhindern, gelöst durch eine Erkennungseinrichtung, die dafür vorgesehen und ausgebildet ist, beim Bohren in einen Knochen mittels einer chirurgischen Bohrmaschine einen Bohrerdurchbruch und/oder eine Durchbruchstendenz zu erkennen, also einer Durchbrucherkennungseinrichtung, mit zumindest einem, insbesondere einachsigen, piezoelektrischen Beschleunigungssensor zur Erzeugung piezoelektrischer Beschleunigungssignale und einer Auswerteeinheit, die dafür vorgesehen und ausgebildet ist, Durchbruchs- und/oder Durchbruchstendenz - charakteristische Signale oder Signalschwankungen in den vom Beschleunigungsssensor ankommenden Signalen zu identifizieren.

In anderen Worten ist eine Erkennungseinrichtung vorgesehen und ausgebildet, in oder an einer chirurgischen Bohrmaschine, vorzugsweise kraftschlüssig, fixiert zu sein. Die Erkennungseinrichtung beinhaltet den piezoelektrischen Beschleunigungssensor. Der piezoelektrische Beschleunigungssensor beinhaltet einen piezoelektrischen Kristall (beispielsweise Quarz) oder eine Keramik, welcher auf einer Seite mit einer trägen seismischen Masse verbunden ist. Wenn (Körper-) Schwingungen der Bohrmaschine auf den piezoelektrischen Beschleunigungssensor wirken, drückt die Masse auf den piezoelektrischen Kristall. Der piezoelektrische Kristall reagiert auf durch die schwingende Masse hervorgerufene Kräfte mit einer Ladungsverschiebung in dem piezoelektrischen Kristall. Auf diese Weise wird die Schwingung in das piezoelektrische Beschleunigungssignal umgewandelt. Das so erzeugte Beschleunigungssignal wird von der Auswerteeinheit ausgewertet. Die Auswerteeinheit erkennt den Durchbruch oder die Durchbruchstendenz anhand der charakteristischen Signale und / oder an den charakteristischen Signalschwankungen.

Bei dem charakteristischen Signal kann es sich beispielsweise um eine maximale Amplitude, eine momentane Amplitude, einen Effektivwert, eine Schwingungsgeschwindigkeit, einen Schwingungsweg oder dergleichen handeln.

In nochmals anderen Worten ist die Erkennungseinrichtung mit der Auswerteeinheit vorgesehen und ausgebildet, einen Materialübergang des Bohrers bzw. eine Materialübergangstendenz des Bohrers und insbesondere einer Bohrerspitze zu erkennen.

Kern der Offenbarung ist demnach, mit der Auswerteeinheit der Erkennungseinrichtung Durchbruchs- und/oder Durchbruchstendenz - charakteristische Signale oder Signalschwankungen, welche von dem piezoelektrischen Beschleunigungssensor der Erkennungseinrichtung aufgrund von Schwingungen der chirurgischen Bohrmaschine erzeugt werden, zu identifizieren.

Durch die derartige Erkennungseinrichtung ist es möglich, zu tiefes Bohren in den Knochen und insbesondere den Durchbruch und / oder die Durchbruchstendenz zu erkennen und damit Schäden an Gewebe, welches sich hinter dem Knochen befindet, zu verhindern. Dies entlastet den operierenden Arzt und erhöht gleichzeitig die Patientensicherheit, da Fehler und / oder Fehleinschätzungen verhindert werden. Zusätzlich können Operationen effizienter durchgeführt werden, da auf eine Analyse von Röntgenbildern eine Bohrtiefe betreffend verzichtet werden kann.

In einem ersten Aspekt kann die Erkennungseinrichtung ausgebildet sein, ein Bohrstoppsignal auszugeben, wenn die Auswerteeinheit die Durchbruchs- und/oder Durchbruchstendenz - charakteristische Signale oder Signalschwankungen identifiziert.

Anders ausgedrückt kann die Erkennungseinrichtung eine Stromzufuhr und/ oder eine (Antriebs-) Luftzufuhr zu einem Antrieb des chirurgischen Bohrers unterbrechen. Alternativ oder zusätzlich kann eine Antriebswelle des chirurgischen Bohrers durch eine Bremse festgesetzt werden.

Weiterhin Alternativ oder zusätzlich kann die Erkennungseinrichtung ausgebildet sein, ein Alarmsignal auszugeben, wenn die Auswerteeinheit die Durchbruchs- und / oder Durchbruchstendenz - charakteristische Signale oder Signalschwankungen identifiziert. Bei dem Alarmsignal kann es sich um ein optisches Signal, beispielsweise in Form einer Anzeige auf einem Display, einem Aufleuchten einer Warnleuchte oder dergleichen, ein akustisches Signal, beispielsweise in Form eines Warntons, und / oder um ein haptisches Signal, beispielsweise in Form einer Vibration, handeln.

Der piezoelektrische Beschleunigungssensor kann Körperschallsignale detektieren, welche als analoge (Beschleunigungs-) Signale an die Auswerteeinheit übermittelt werden.

In anderen Worten kann der piezoelektrische Beschleunigungssensor Schwingungen und Wellen, welche sich in Festkörpern mit endlicher Geschwindigkeit ausbreiten, detektieren und in das analoge Signal umwandeln. Bei dem Festkörper handelt es sich insbesondere um die Bohrmaschine oder um Komponenten der Bohrmaschine und / oder um den Bohrer. Das analoge Signal wird anschließend, vorzugsweise mit einem Kabel oder dergleichen, an die Auswerteeinheit übermittelt.

Durch die Detektion von Körperschall kann ein Einfluss von Umwelteinflüssen, insbesondere in Form von Geräuschen in einem Operationssaal, beispielsweise Piepen von Maschinen, Stimmen, durch Bewegungen verursachte Geräusche, bis hin zu Lärm, auf das Erkennungsergebnis reduziert werden. Auf diese Weise kann gerade in besonders hektischen Situationen im Operationssaal der operierende Arzt unterstützt werden und zu tiefes Bohren effektiv verhindert werden und der Arzt kann sich auf das Erkennungsergebnis, unabhängig von der Umgebung in der er sich befindet, verlassen.

**In** einem weiteren Aspekt kann die Auswerteeinheit der Erkennungseinrichtung zumindest einen Verstärker insbesondere mit einer Verstärkung größer / gleich zehn und / oder einen Filter, insbesondere ein Butterworth-Filter n-ter Ordnung oder ein Bandpassfilter, beinhalten.

In anderen Worten kann das analoge Signal, welches von dem piezoelektrischen Beschleunigungssensor ausgegeben wird, von der Auswerteeinheit mit einem in der Auswerteeinheit vorgesehenen Verstärker verstärkt und / oder von einem in der Auswerteeinheit vorgesehenen Filter gefiltert werden. In nochmals anderen Worten kann der Verstärker das analoge Signal in eine proportionale Spannung umwandeln und sie verstärken. Eine anschließende Hoch- oder Tiefpassfilterung kann störende Signalanteile unterdrücken / herausfiltern.

In einem weiteren Aspekt kann die Auswerteeinheit einen Analog-Digital-Wandler beinhalten, welcher die analogen Signale mit einer Abtastrate von zumindest 20000 Hz, insbesondere mit einer Abtastrate von ungefähr 25600 Hz abtastet.

In anderen Worten kann ein "Sampling" des analogen Signals stattfinden. In nochmals anderen Worten kann eine Amplitude einer Wellenform des analogen Signals zu verschiedenen (definierten) Zeitpunkten ausgelesen werden. Diese diskreten Amplitudenwerte werden in einem Array oder in einem Vektor gespeichert. Nach dem Nyquist-Shannon-Theorem muss die Abtastrate / eine Abtastfrequenz des analogen Signals mehr als doppelt so groß sein wie die höchste Frequenz des zu digitalisierenden Signals. Andernfalls kann es zu einem Aliasingeffekt kommen. Durch das Digitalisieren kann eine Signalauswertung verbessert werden.

**In** nochmals anderen Worten können der Analog-Digital-Wandler und der piezoelektrische Beschleunigungssensor, insbesondere in der Abtastrate, aufeinander abgestimmt sein.

**In** einem weiteren Aspekt kann die Auswerteeinheit, vorzugsweise mit einem Microcontroller, das Signal bzw. die Signale mittels einer Fouriertransformation, insbesondere einer Short-Time-Fourier-Transformation, in ein Spektrogramm, vorzugsweise in ein Melspektrogramm, umwandeln.

In anderen Worten kann die Auswerteeinheit vorzugsweise mit einem dafür vorgesehenen Microcontroller das Signal, welches vorzugsweise in Form eines kontinuierlichen Zeitsignals vorliegt, in seine diskreten Frequenzanteile zerlegen. In der Short-Time-Fourier-Transformation können hierbei auch Signale verarbeitet werden, deren Frequenzeigenschaften sich mit der Zeit verändern. Hierbei wird zunächst ein Fenster und dessen Parameter Fenstergröße und Schrittgröße festgelegt. Dieses Fenster wird über das Signal geschoben. Für jedes Fenster wird die Fouriertransformation berechnet und ein Spektrum gebildet. Durch Verbinden der Spektren entsteht ein Spektrogramm. Bei einem Spektogramm handelt es sich um eine Überlagerung einer Folge von Spektren in einem vorbestimmten Zeitintervall. Aus einem Spektrogramm lässt sich somit die Verteilung der Frequenzen und ihrer Intensität während des Verlaufs über der Zeit ablesen. Durch eine derartige Umwandlung des Signals kann das Signal optisch auswertbar gemacht werden.

In einem weiteren Aspekt kann die Auswerteeinheit das Spektrogramm mit einem Kl-trainierten Netzwerk, vorzugsweise mittels eines "bounding boxes-Prinzip" auswerten und bei einem Aufleuchten des Spektrogramms den Bohrerdurchbruch oder die Bohrerdurchbruchstendenz identifizieren.

In anderen Worten kann die Auswerteeinheit das Spektrogramm mit einem Netzwerk, welches mittels Maschinenlernen trainiert wird, auswerten. Hierbei wird das Spektogramm bevorzugt optisch ausgewertet. Insbesondere kommt das "bounding boxes-Prinzip" zu Einsatz. Bei dem "bounding box-Prinzip" wird zumindest ein Rechteck, bevorzugt mehrere Rechtecke, welche jeweils mit einem Label versehen sind, über das Spektrogramm gelegt. Jedes der Rechtecke wird über die Koordinaten der oberen linken Ecke und die Koordinaten der unteren rechten Ecke charakterisiert. Bei dem Label kann in drei Klassen unterschieden werden. Die erste Klasse ist dabei "Leerlauf", die zweite Klasse ist "Bohren" und die dritte Klasse ist "Durchbruch". Es kann eine weitere Klasse Null für den Hintergrund verwendet werden. In der Klasse "Leerlauf" ist ausschließlich eine Grundfrequenz zu erkennen. In der Klasse "Bohren" ist eine Frequenzaufspaltung zu erkennen. In der Klasse "Durchbruch" kehrt das Spektrogramm wieder in annähernd in einen Leerlaufzustand zurück, wobei noch Oberwellen enthalten sind, da der Bohrer den Knochen noch nicht vollständig verlassen hat. Das Rechteck, welches mit "Durchbruch" gelabelt ist, beginnt vor dem eigentlichen Durchbrechen des Bohrers. Als Indikator für einen Durchbruch und / oder eine Durchbruchstendenz wird insbesondere ein Aufleuchten in zumindest einer Oberwelle zur Grundfrequenz verwendet. Das Aufleuchten in dem Spektrogramm kommt durch ein vermehrtes Auftreten von hohen Amplituden im Signal zustande. Das Netzwerk kann anhand von Trainerdaten trainiert werden. Weiterhin kann das Netzwerk fortlaufend weiter trainiert werden und damit die Identifikation über die Nutzung des chirurgischen Bohrers mit der erfindungsgemäßen Erkennungseinrichtung verbessert werden.

Die optische Analyse durch das "boundig box-Prinzip" ist ein besonders robustes Analyseverfahren. Durch das Netzwerk, welches auf Basis von Trainerdaten trainiert ist und während der Verwendung fortlaufend weiter lernt, kann eine zuverlässige Identifikation von dem Durchbruch und / oder der Durchbruchstendenz erzielt werden.

Die Spektrogramme stellen eine Überlagerung vieler Fouriertransformationen dar und liefern genaue Informationen über die Frequenz und die Größe der Amplituden bei Durchbruch. Diese Merkmale werden durch die KI erlernt, darunter auch die Werte für die Aufspreizung des Frequenzspektrums bei Durchbruch. Daraus ergeben sich Werte von +/- einer Veränderung der Grundfrequenz bzw. deren Harmonischen und der Drehzahl. Da die Grundfrequenz und die jeweilige Drehzahl der Bohrer bekannt sind, können Veränderungen in der Fouriertransformation erkannt werden, solang diese im durch die KI definierten Bereich liegen.

In einem weiteren Aspekt kann das KI-trainierte Netzwerk ein Cloudnetzwerk sein, das von kompatiblen Erkennungseinrichtungen mit Daten gespeist wird.

In anderen Worten kann das Kl-trainierte Netzwerk ein Netzwerk sein, dass von einer Vielzahl kompatibler Erkennungseinrichtungen für die Erkennung / Identifikation des Durchbruchs und / oder der Durchbruchstendenz verwendet wird.

Auf diese Weise kann eine rechenintensive Analyse des Spektrogramms zentralisiert durch einen entsprechenden Rechner erfolgen und damit Investitionskosten vor Ort, beispielsweise im Krankenhaus, reduziert werden. Ferner kann durch eine Bündelung einer Vielzahl von Daten von der Vielzahl von kompatiblen Erkennungseinrichtungen die Identifikation des Durchbruchs und / oder der Durchbruchstendenz erheblich verbessert werden. Zusätzlich bietet das Cloudnetzwerk den Vorteil, dass eine Redundanz wirtschaftlich sinnvoll umsetzbar ist, was eine Ausfallsicherheit erhöht.

In einem weiteren Aspekt kann der piezoelektrische Beschleunigungssensor seine höchste Empfindlichkeit in einem Messfrequenzbereich bis 10000 Hz besitzen.

Weiterhin wird die Aufgabe, zu tiefes Bohren in den Knochen zu verhindern, gelöst durch eine chirurgische Bohrmaschine, in oder an welcher die Erkennungseinrichtung nach einem der obigen Aspekte ausgebildet ist.

In anderen Worten ist die Erkennungseinrichtung, die dafür vorgesehen und ausgebildet ist, beim Bohren in den Knochen mittels der chirurgischen Bohrmaschine den Bohrerdurchbruch und/oder die Durchbruchstendenz zu erkennen, mit, zumindest dem zumindest einen, insbesondere einachsigen, piezoelektrischen Beschleunigungssensor zur Erzeugung piezoelektrischer Beschleunigungssignale und der Auswerteeinheit, die dafür vorgesehen ist, die Durchbruchs- und/oder Durchbruchstendenz - charakteristische Signale oder Signalschwankungen zu identifizieren in oder an der chirurgischen Bohrmaschine ausgebildet.

In einem Aspekt kann der piezoelektrische Beschleunigungssensor durch direkte metallische Anbindung an einen Antrieb der chirurgischen Bohrmaschine gekoppelt sein.

In anderen Worten kann der piezoelektrische Beschleunigungssensor unmittelbar oder mittels zumindest eines vorzugsweise metallischen Halters mechanisch mit dem Antrieb der chirurgischen Bohrmaschine verbunden sein. Bei dem Antrieb kann es sich beispielsweise um einen Elektromotor oder um eine Turbine handeln. Neben einer metallischen Ausbildung des Halters ist jeder andere Werkstoff vorstellbar, der Körperschall möglichst verlustarm und ungedämpft leitet und dabei den Werkstoffanforderungen einer chirurgischen Bohrmaschine genügt.

Durch die direkte Anbindung ist gewährleistet, dass der piezoelektrische Beschleunigungssensor bereits geringe Beschleunigungen und / oder Änderungen in der Beschleunigung detektieren kann. Weiterhin kann auf diese Weise ein Einfluss von, von außerhalb der chirurgischen Bohrmaschine eingebrachter Schwingungen, beispielsweise durch Lärm, zuverlässig gefiltert werden.

In einem weiteren Aspekt kann / können ein Verstärker und / oder ein Filter der Auswerteeinheit auf einer Platine ausgebildet sein, welche in direkter Nähe zu dem piezoelektrischen Beschleunigungssensor, vorzugsweise hermetisch abgedichtet, in / an der chirurgischen Bohrmaschine ausgebildet ist.

Anders ausgedrückt kann in / an der chirurgischen Bohrmaschine eine vorzugsweise hermetisch abgedichtete Leiterplatte mit Bauteilen ausgebildet sein, wobei die Bauteile den Filter und / oder den Verstärker ausbilden können. Die Leiterplatte kann in unmittelbarer Nähe zu dem piezoelektrischen Beschleunigungssensor ausgebildet sein. Die Verstärkung/Verstärkerleistung des Verstärkers kann vorzugsweise mindestens zehn betragen. Der Filter kann die Signale auf den gewünschten Signalbereich beschränken und kann unabhängig gegenüber Störsignalen sein, welche beispielsweise von dem Bohrer und seiner Rotation selbst verursacht werden.

Durch die direkte Nähe zwischen piezoelektrischem Beschleunigungssensor und dem Verstärker und / oder dem Filter werden Verluste bei einer Übertragung von dem piezoelektrischen Beschleunigungssensor zu dem Verstärker und / oder dem Filter minimiert. Auf diese Weise kann das Erkennungsergebnis der Erkennungseinrichtung verbessert werden.

In einem weiteren Aspekt kann die Auswerteeinheit vollständig oder teilweise in einer extern von der chirurgischen Bohrmaschine vorgesehenen Basiseinheit (Steuergerät) ausgebildet sein.

In anderen Worten kann der Filter und / oder der Verstärker in / an der chirurgischen Bohrmaschine / einem Handstück der chirurgischen Bohrmaschine ausgebildet sein. Weitere Komponenten, wie beispielsweise ein Analog-Digital-Wandler und ein oder mehrere Microcotroller können außerhalb des Handstücks der chirurgischen Bohrmaschine in der Basiseinheit ausgebildet sein. Das Handstück und die Basiseinheit können mit einem Kabel und / oder einem Schlauch miteinander verbunden sein. Die Basiseinheit kann Komponenten, welche in dem Handstück ausgebildet sind, über das Kabel mit elektrischer Energie versorgen.

Auf diese Weise kann das Handstück der chirurgischen Bohrmaschine besonders kompakt gestaltet werden, was eine Handhabung für den Behandler erleichtert. Insbesondere wenn der Filter und / oder der Verstärker in / an dem Handstück ausgebildet sind, kann gewährleistet werden, dass die Rohsignale des piezoelektrischen Beschleunigungssensors in einer geeigneten Form (verstärkt, gefiltert) an die Basiseinheit übermittelt werden. In der Basiseinheit können die Signale dann durch den Analog-Digital-Wandler abgetastet werden und anschließend durch einen Microcontroller, vorzugsweise mittels Short-Time-Fourier-Transformation, in das Spektrogramm umgewandelt werden. Diese Spektrogramme werden anschließend, vorzugsweise durch eine in der Basiseinheit ausgebildeten Kommunikationseinheit an das Kl-trainierte Netzwerk übermittelt. Das Kl-trainierte Netzwerk kann cloudbasiert sein. Alternativ kann das Spektrogramm in der Basiseinheit vorzugsweise mittels weiterer Microcontroller mit Speicher- und Datenverarbeitungsfunktion ausgewertet werden. Auch kann auf diese Weise ein modularer Aufbau umgesetzt werden, sodass mehrere Handstücke mit einer Basiseinheit ausgebildet sein können. Dies reduziert die Kosten und erweitert einen Behandlungsumfang durch eine unterschiedliche, an die Behandlung angepasste Handstücke.

In einem weiteren Aspekt können mehrere piezoelektrische Beschleunigungssensoren an dem Handstück der chirurgischen Bohrmaschine ausgebildet sein.

In einem weiteren Aspekt können die mehreren piezoelektrischen Beschleunigungssensoren distanziert voneinander in dem Handstück der chirurgischen Bohrmaschine ausgebildet sein.

Weiterhin wird die Aufgabe ein Verfahren bereitzustellen, welches vorbestimmte Betriebszustände einer chirurgischen Bohrmaschine zuverlässig erkennt, durch das Verfahren zur Erkennung vorbestimmter Betriebszustände einer chirurgischen (Knochen-) Bohrmaschine mit den folgenden Verfahrensschritten gelöst:
- Erzeugen von piezoelektrischen Beschleunigungssignalen unter Verwendung eines, insbesondere einachsigen, piezoelektrischen Beschleunigungssensors, der an oder in der Knochenbohrmaschine angeordnet ist oder wird,
- Weiterleiten der Beschleunigungssignale an eine Auswerteeinheit und
- Identifizieren von solchen Signalen oder Signalschwankungen, die für die vorbestimmten Betriebszustände charakteristisch sind.

Durch das beschriebene Verfahren ist es möglich, Fehlfunktionen der chirurgischen Bohrmaschine frühzeitig zu erkennen. So kann beispielsweise ein Lagerschaden in der Lagerung des Bohrers der chirurgischen Bohrmaschine detektiert werden. Weiterhin kann ein Unrundlaufen des Bohrers, beispielsweise durch falsches Einspannen des Bohrers in der chirurgischen Bohrmaschine, erkannt werden. Bei einer erkannten Fehlfunktion kann die chirurgische Bohrmaschine gestoppt und / oder ein akustisches oder haptisches Alarmsignal ausgegeben werden. Bei den vorbestimmten Betriebszuständen kann es sich beispielsweise um einen Normalzustand und einen Störungszustand oder einen Materialübergang handeln.

In einem Aspekt kann in der Auswerteeinheit das Beschleunigungssignal ausgewertet werden mit den Schritten:
- Filtern des Beschleunigungssignals mit einem Filter, vorzugsweise einem Butterworth-Filter n-ter Ordnung oder einem Bandpassfilter;
- Verstärken des Beschleunigungssignals mit einem Verstärker, vorzugsweise mit einem Verstärker mit einer Verstärkung größer / gleich 10;
- Abtasten des Beschleunigungssignals mit einem Analog-Digital-Wandler;
- Umwandeln des (digitalen) Beschleunigungssignals in Spektrogramme, vorzugsweise mittels Fouriertransformation, insbesondere vorzugsweise mittels Short-Time-Fourier-Transformation;
- Auswerten der Spektrogramme mittels eines "Bounding boxes-Prinzip" in einem KI-Trainierten Netzwerk.

Weiterhin betrifft die vorliegende Offenbarung eine Verwendung eines insbesondere einachsigen, piezoelektrischen Beschleunigungssensors, der zur Erfassung vorbestimmter Betriebszustände an oder in einer Knochenbohrmaschine angeordnet ist oder wird.

An dieser Stelle sei nochmals abschließend darauf hingewiesen, dass die Identifikation von solchen Signalen oder Signalschwankungen, die für die vorbestimmten Betriebszustände charakteristisch sind, beispielhaft auch dadurch erfolgen könnte, indem in der einfachsten Ausführung Signalmuster oder Schwankungsmuster mit vorab bereits hinterlegten, bestimmten Mustern auf Übereinstimmungen verglichen werden. Alternativ oder zusätzlich wäre es auch denkbar, absolute oder relative Signalwerte/Amplituden/Tastverhältnisse/Signaldauern etc. bestimmten Betriebszuständen zuzuordnen. Letztlich ist es grundsätzlich möglich, Erkennungsalgorythmen vergleichbar zu Bilderkennungsalgorythmen bereitzustellen.

### Kurzbeschreibung der Figuren

Fig. 1 ist eine Darstellung einer chirurgischen Bohrmaschine mit einem ersten Handstück und einer Basiseinheit;
Fig. 2 ist eine Darstellung eines zweiten Handstücks;
Fig. 3 ist eine vergrößerte Darstellung des zweiten Handstücks;
Fig. 4 ist eine Darstellung einer Hülse für ein erstes Handstück;
Fig. 5 ist eine Darstellung eines piezoelektrischen Beschleunigungssensors.
Fig. 6 zeigt einen Graphen einer detektierten Schwingung und ein Spektrogramm.

### Beschreibung der Ausführungsbeispiele

Nachstehend werden Ausführungsbeispiele der vorliegenden Offenbarung auf der Basis der zugehörigen Figuren beschrieben.

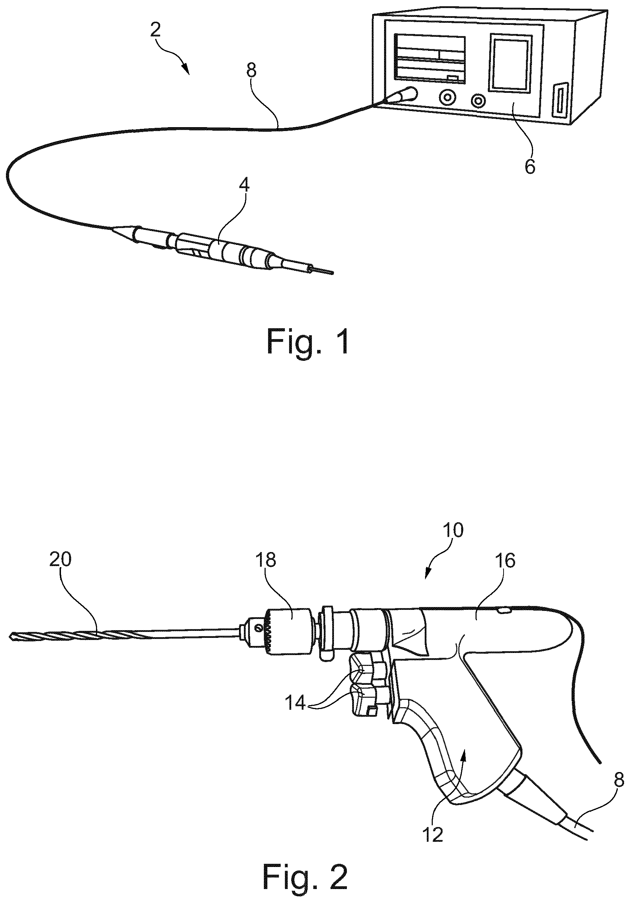

Fig. 1 zeigt eine chirurgische Bohrmaschine 2 mit einem ersten Handstück 4 in einer erste Ausführungsform und einer Basiseinheit 6. Das erste Handstück 4 ist mit einem Kabel 8 mit der Basiseinheit 6 verbunden.

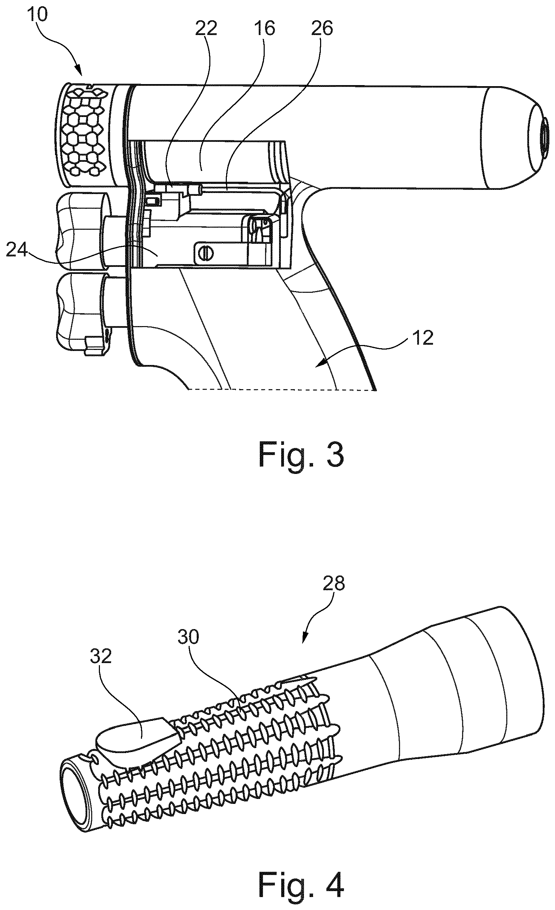

Fig. 2 zeigt ein zweites Handstück 10 in einer zweiten Ausführungsform. Fig. 3 zeigt einen Ausschnitt des zweiten Handstücks 10 in einer vergrößerten Ansicht. Nachfolgend wird das zweite Handstück anhand von Fig. 2 und Fig.3 näher erläutert. Das zweite Handstück 10 ist mit dem Kabel 8 mit der Basiseinheit 6 verbunden. Das zweite Handstück 10 beinhaltet einen Griffabschnitt 12 in Form eines Pistolengriffs, der vorgesehen und ausgebildet ist, von einem Behandler, beispielsweise einem Arzt umgriffen zu werden, so dass das zweite Handstück 10 sicher geführt werden kann. An einer Vorderseite des Griffabschnitts 12 sind Bedienknöpfe 14 ausgebildet. Mit den Bedienknöpfen 14 kann beispielsweise eine Rotationsrichtung und eine Rotationsgeschwindigkeit der chirurgischen Bohrmaschine bedient werden. An einem von dem Kabel 8 abgewandten Ende des Griffabschnitts 12 ist ein elektrischer Antrieb 16 ausgebildet. Anders ausgedrückt ist der elektrische Antrieb an einer Oberseite des zweiten Handstücks 10, oberhalb des Griffabschnitts 12, ausgebildet. An einer Vorderseite des elektrischen Antriebs 16 ist ein Bohrfutter 18 ausgebildet, das vorgesehenen ausgebildet ist einen Bohrer 20 aufzunehmen. Das Bohrfutter 18 ragt dabei über den Griffabschnitt 12 und die Bedienknöpfe 14 hinaus. Der Antrieb 16 und das Bohrfutter 18 sind vorzugsweise direkt oder mittels eines Getriebes (nicht dargestellt) verbunden. Direkt mit dem Antrieb 16 verbunden ist ein piezoelektrischer Beschleunigungssensor 22 ausgebildet. Anders ausgedrückt ist der piezoelektrische Beschleunigungssensor 22 direkt metallisch mit dem Antrieb 16 gekoppelt. In nochmals anderen Worten kontaktiert der piezoelektrische Beschleunigungssensor 22 den Antrieb 16 direkt. Alternativ ist vorstellbar, dass der piezoelektrische Beschleunigungssensor 22 mittels eines metallischen Verbindungselements mit dem Antrieb 16 verbunden ist. Auf diese Weise kann ein Körperschall der chirurgischen Bohrmaschine und insbesondere Körperschall des Antriebs 16 direkt von dem piezoelektrischen Beschleunigungssensor 22 aufgenommen werden. Der piezoelektrische Beschleunigungssensor 22 leitet detektiere Beschleunigungssignale an einen Filter und einen Verstärker weiter. Der Filter und der Verstärker sind in einer hermetisch gekapselten Leiterplatte 24 ausgebildet. Der piezoelektrische Beschleunigungssensor 22 und die gekapselte Leiterplatte 24 sind hierbei in unmittelbarer Nähe zueinander in dem Handstück 10 der chirurgischen Bohrmaschine 2 untergebracht. Der piezoelektrische Beschleunigungssensor 22 und die gekapselte Leiterplatte 24 sind mit einem Sensorkabel 26 verbunden. Der piezoelektrische Beschleunigungssensor 22 und die gekapselte Leiterplatte 24 sind in dem hier dargestellten Ausführungsbeispiel vollständig in dem Griffabschnitt 12 des Handstücks 10 aufgenommen.

Fig. 4 zeigt eine alternative Ausführungsform, insbesondere für ein erstes Handstück 4, einer ersten Ausführungsform. Konkret zeigt Fig. 4 eine Haltehülse 28, welche vorgesehen und ausgebildet ist, an einem ersten Handstück 4 angebracht zu werden und einen Bohrer 20 in einer Längsrichtung der Haltehülse 28, insbesondere zentrisch aufzunehmen und gegebenenfalls zu führen. Die Haltehülse 28 beinhaltet einen geriffelten Halteabschnitt 30 und einen Befestigungsabschnitt 32. Der Befestigungsabschnitt 32 ist vorgesehen und ausgebildet, den piezoelektrischen Beschleunigungssensor 22 (in dieser Ausführungsform nicht dargestellt) aufzunehmen. Der piezoelektrische Beschleunigungssensor 22 kann insbesondere mittels einer Fixierungsmasse oder einem Klebstoff oder einem Rastmechanismus oder dergleichen Fixiermittel auf dem Befestigungsabschnitt 32 der Haltehülse 28 fixiert werden. In anderen Worten kann der piezoelektrische Beschleunigungssensor 22 stoffschlüssig oder kraftschlüssig an der Haltehülse 28 fixiert werden. Das Hauptaugenmerk liegt hierbei auf einer direkten Verbindung zwischen piezoelektrischem Beschleunigungssensor 22 und der Haltehülse 28. Anders ausgedrückt ist der piezoelektrische Beschleunigungssensor 22 so auf oder an der Haltehülse 28 fixiert, dass Schwingungen der Haltehülse 28 möglichst direkt und ohne Dämpfung an den piezoelektrischen Beschleunigungssensor 22 weitergeleitet werden. Im Unterschied zu der zweiten Ausführungsform in Fig. 2 und Fig. 3 ist der piezoelektrische Beschleunigungssensor 22 in der ersten Ausführungsform außerhalb des ersten Handstücks 4 ausgebildet. Bevorzugt ist bei dieser Ausführungsform der piezoelektrische Beschleunigungssensor 22 und die gekapselte Leiterplatte 24 in einem geschlossenen Identifikationselement /nicht dargestellt) ausgebildet. Die in Fig. 4 dargestellte Ausführungsform ist beispielsweise auch als eine Nachrüstlösung vorstellbar.

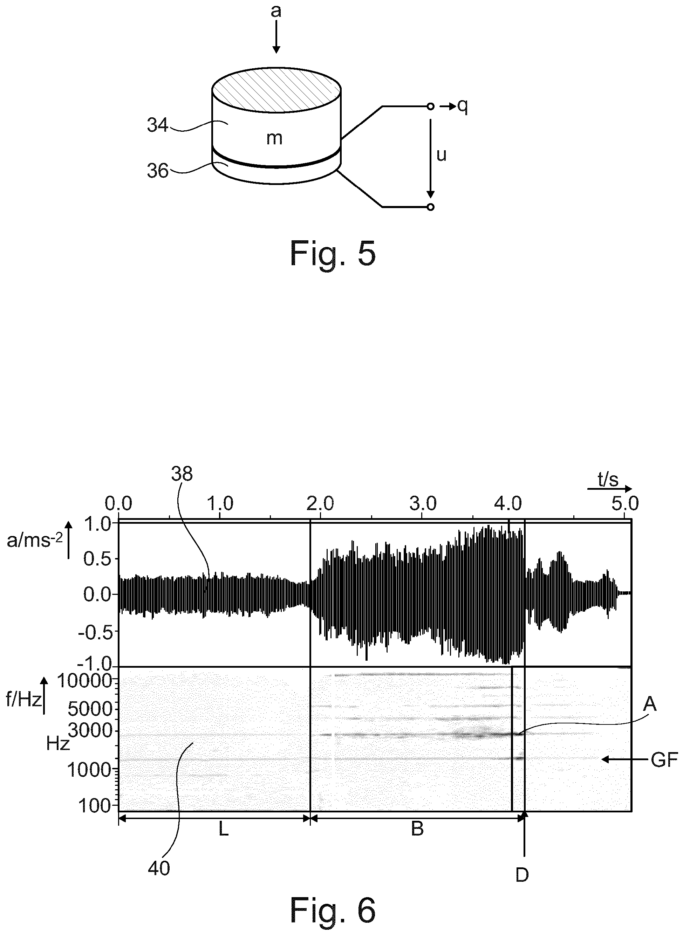

Fig. 5 zeigt einen piezoelektrischen Beschleunigungssensor 22 in einer schematischen Darstellung. Der piezoelektrische Beschleunigungssensor 22 beinhaltet eine seismische Masse 34 und einem Piezokristall / Piezokeramik 36. Der piezoelektrische Beschleunigungssensor 22 ist vorgesehen und ausgebildet, eine mechanische Schwingungsbeschleunigung a, welche auf den piezoelektrischen Beschleunigungssensor 22 wirkt, in ein Spannungsmesssignal U umzuwandeln. Anders ausgedrückt wirkt eine Schwingung auf den piezoelektrischen Beschleunigungssensor 22 und drückt die träge seismische Masse 34 auf den Piezokristall 36. Piezoelemente wie der Piezokristall 36 reagieren auf einwirkende Kräfte mit einer Ladungsverschiebung q im Kristall. Auf diese Weise wird die auf den piezoelektrischen Beschleunigungssensor 22 wirkende Schwingungsbeschleunigung in eine elektrische Ladung transformiert. Diese elektrische Ladung ist proportional zu einer einwirkenden Kraft. Die seismische Masse 34 und der Piezokristall 36 bilden ein Feder-Masse-System mit Tiefpassverhalten und linearen Frequenzbereich.

Im Folgenden wird die Funktionsweise der Erkennungseinrichtung der chirurgischen Bohrmaschine 2 erläutert. Bei dem Bohren in Knochen entstehen durch die Interaktion zwischen dem Bohrer 20 und dem Knochen Schwingungen in der chirurgischen Bohrmaschine 2. Diese Schwingungen entstehen insbesondere in dem Antrieb 16 der chirurgischen Bohrmaschine 2. Der piezoelektrische Beschleunigungssensor 22 detektiert die Schwingungen in dem Antrieb 16 und überträgt ein analoges Spannungssignal mit dem Sensorkabel 26 an den Filter und den Verstärker, die sich auf der gekapselten Leiterplatte 24 befinden. Das analoge Spannungssignal wird von dem Filter gefiltert, um störende Signalteile herauszufiltern. Dies geschieht beispielsweise mit einer Hochpass- oder Tiefpassfilterung. Im Verstärker wird das Signal vorzugsweise um mindestens den Faktor zehn verstärkt. Das so vorbehandelte Signal (38 in Fig. 6) wird über das Kabel 8, welches das Handstück 10 mit der Basiseinheit 6 verbindet, an die Basiseinheit 6 übertragen.

Nachfolgend wird anhand von Fig. 6 eine Auswertung des vorbehandelten Signals 38 beschrieben.

Das durch den Filter und den Verstärker der gekapselten Leiterplatte 24 vorbehandelte Signal 38 liegt in einer WAV-Form vor. Das Signal 38 bildet die von dem piezoelektrischen Beschleunigungssensor detektierte Beschleunigung über der Zeit t, hier in Sekunden ab. Das Signal wird in der Basiseinheit 6 von einem in der Basiseinheit 6 ausgebildeten Analog-digital-Wandler abgetastet und anschließend mit einem Mikrocontroller in Frequenzanteile zerlegt. Die Zerlegung in die Frequenzanteile erfolgt, indem über definierte Zeitfenster eine Short-Time-Fouriertransformation durchgeführt wird. Ein erhaltenes Endsignal in Form eines Spektrogramms 40 repräsentiert die Zeit t, hier in Sekunden, auf der Abszisse zur Frequenz f, Hier in Hertz, auf der Ordinate mit der Amplitude als Helligkeit. Zur besseren Veranschaulichung in dieser Offenbarung ist das Spektrogramm 40 invertiert dargestellt. Anders ausgedrückt sind Bereiche, die in dem eigentlichen Spektrogramm dunkel sind, hier hell dargestellt und helle Bereiche in dem eigentlichen Spektrogramm sind in dem hier dargestellten Spektrogramm 40 dunkel dargestellt. Das Spektrogramm 40 wird anschließend über in der Basiseinheit 6 enthaltene Mikrocontroller bzw. über ein KI trainiertes Netzwerk ausgewertet. Die Auswertung erfolgt nach dem "bounding box-Prinzip". In anderen Worten wird das Spektrogramm 40 grafisch ausgewertet. Das KI trainierte Netzwerk legt unterschiedlich gelabelte Rechtecke über das Spektrogramm 40. Das KI trainierte Netzwerk positioniert die Rechtecke beispielsweise anhand der Helligkeit im Spektrogramm 40. Als Label werden "Leerlauf L", "Bohren B" und "Durchbruch D" verwendet. Die Rechtecke können sich hierbei auch überlappen.

Die Detektion des Durchbruchs und / oder der Durchbruchstendenz erfolgt durch das KI trainierte Netzwerk auf Basis eines Aufleuchtens A im Spektrogramm 40. Wie oben bereits beschrieben, ist das Aufleuchten A in der vorliegenden Offenbarung zur besseren Veranschaulichung farbinvertiert dargestellt. Im Spektrrogramm 40 ist auch die Grundfrequenz GF des Handstücks 10 deutlich zu erkennen. Das Aufleuchten A im Spektrogramm 40 steht für ein vermehrtes Auftreten hoher Amplituden im Signal. Das KI trainierte Netzwerk ist darauf trainiert, solche optischen Veränderungen in dem Spektrogramm 40 zu erkennen. Das KI trainierte Netzwerk kann bei der Erkennung eines Durchbruchs beispielsweise einen Motorstoppbefehl oder einen Alarm ausgeben. Bei dem Alarm kann es sich um einen akustischen Alarm in Form eines Warntones oder um einen optischen Alarm in Form eines Blinklichts oder dergleichen oder um einen haptischen Alarm in Form einer Vibration des Handstücks 4, 10 handeln.

Zusammenfassend detektiert der piezoelektrische Beschleunigungssensor 22 Schwingungsbeschleunigungen des Antriebs 16 des chirurgischen Bohrers 2. Diese Schwingungsbeschleunigungen werden durch einen Filter und ein Verstärker in oder an dem Handstück 4, 10 vorverarbeitet, und anschließend von einem Analog-digital-Wandler in der Basiseinheit 6 abgetastet. Nachfolgend werden die Signale von dem Mikrocontroller in das Spektrogramm 40 umgewandelt. Das Spektrogramm 40 wird abschließend von einem KI trainierten Netzwerk analysiert / ausgewertet und anhand von optischen Veränderungen des Spektrogramm 40 wird ein Durchbruch bzw. eine Durchbruchstendenz erkannt.

### Bezugszeichenliste

- 2: chirurgische Bohrmaschine
- 4: erstes Handstück
- 6: Basiseinheit
- 8: Kabel
- 10: zweites Handstück
- 12: Griffabschnitt
- 14: Bedienknöpfe
- 16: Antrieb
- 18: Bohrfutter
- 20: Bohrer
- 22: piezoelektrischer Beschleunigungssensor
- 24: Leiterplatte
- 26: Sensorkabel
- 28: Haltehülse
- 30: Halteabschnitt
- 32: Befestigungsabschnitt
- 34: seismische Masse
- 36: Piezokristall
- 38: (vorbehandeltes) Signal
- 40: Spektrogramm
- L: Leerlauf
- B: Bohren
- D: Durchbruch
- A: Aufleuchten
- GF: Grundfrequenz
- t: Zeit
- f: Frequenz

## Patentansprüche

1. Chirurgische Bohrmaschine (2), in oder an welcher eine Erkennungseinrichtung ausgebildet ist, wobei die Erkennungseinrichtung, dafür vorgesehen und ausgebildet ist, beim Bohren in einen Knochen mittels der chirurgischen Bohrmaschine (2) einen Bohrerdurchbruch und/oder eine Durchbruchstendenz zu erkennen, mit zumindest einem, insbesondere einachsigen, piezoelektrischen Beschleunigungssensor (22) zur Erzeugung piezoelektrischer Beschleunigungssignale und einer Auswerteeinheit, die dafür vorgesehen ist, Durchbruchs- und/oder Durchbruchstendenz - charakteristische Signale oder Signalschwankungen zu identifizieren, **dadurch gekennzeichnet, dass** der piezoelektrische Beschleunigungssensor (22) Körperschallsignale detektiert, welche als analoge Signale an die Auswerteeinheit übermittelt werden.

2. Chirurgische Bohrmaschine (2) nach Anspruch 1, wobei die Auswerteeinheit zumindest einen Verstärker, insbesondere mit einer Verstärkung größer / gleich zehn und / oder einen Filter, insbesondere ein Butterworth-Filter n-ter Ordnung oder ein Bandpassfilter, beinhaltet.

3. Chirurgische Bohrmaschine (2) nach Anspruch 2, wobei die Auswerteeinheit einen Analog-Digital-Wandler beinhaltet, welcher die analogen Signale mit einer Abtastrate von zumindest 20000 Hz, insbesondere mit einer Abtastrate von ungefähr 25600 Hz abtastet.

4. Chirurgische Bohrmaschine (2) nach Anspruch 2 oder 3, wobei die Auswerteeinheit, vorzugsweise mit einem Microcontroller, die Signale mittels einer Fouriertransformation, insbesondere einer Short-Time-Fourier-Transformation, in ein Spektrogramm (40), vorzugsweise in ein Melspektrogramm, umwandelt.

5. Chirurgische Bohrmaschine (2) nach Anspruch 4, wobei die Auswerteeinheit das Spektrogramm (40) mit einem Kl-trainierten Netzwerk, vorzugsweise mittels eines "Bounding boxes-Prinzip" auswertet und bei einem Aufleuchten des Spektrogramms (40) den Bohrerdurchbruch oder die Bohrerdurchbruchstendenz identifizieret.

6. Chirurgische Bohrmaschine (2) nach Anspruch 5, wobei das KI-trainierte Netzwerk ein Cloudnetzwerk ist, das von kompatiblen Erkennungseinrichtungen mit Daten gespeist wird.

7. Chirurgische Bohrmaschine (2) nach einem der Ansprüche 1 bis 6, wobei der piezoelektrische Beschleunigungssensor (22) seine höchste Empfindlichkeit in einem Messfrequenzbereich bis 10000 Hz besitzt.

8. Chirurgische Bohrmaschine (2) nach einem der Ansprüche 1 bis 7, wobei der piezoelektrische Beschleunigungssensor (22) durch direkte metallische Anbindung an einen Antrieb (16) der chirurgischen Bohrmaschine (2) gekoppelt ist.

9. Chirurgische Bohrmaschine (2) nach einem der Ansprüche 1 bis 8, wobei ein Verstärker und / oder ein Filter der Auswerteeinheit auf einer Platine 24 ausgebildet ist / sind, welche in direkter Nähe zu dem piezoelektrischen Beschleunigungssensor 22, vorzugsweise hermetisch abgedichtet, in / an der chirurgischen Bohrmaschine (2) ausgebildet sind.

10. Chirurgische Bohrmaschine (2) nach einem der Ansprüche 1 bis 9, wobei eine Auswerteeinheit vollständig oder teilweise in einer extern von der chirurgischen Bohrmaschine vorgesehenen Basiseinheit (6) ausgebildet ist.

11. Erkennungseinrichtung, die dafür vorgesehen und ausgebildet ist, beim Bohren in einen Knochen mittels einer chirurgischen Bohrmaschine (2) einen Bohrerdurchbruch und/oder eine Durchbruchstendenz zu erkennen, mit zumindest einem, insbesondere einachsigen, piezoelektrischen Beschleunigungssensor (22) zur Erzeugung piezoelektrischer Beschleunigungssignale und einer Auswerteeinheit, die dafür vorgesehen ist, Durchbruchs- und/oder Durchbruchstendenz - charakteristische Signale oder Signalschwankungen zu identifizieren, **dadurch gekennzeichnet, dass** der piezoelektrische Beschleunigungssensor (22) Körperschallsignale detektiert, welche als analoge Signale an die Auswerteeinheit übermittelt werden.

## Claims

1. Surgical drill (2) in or on which a detection device is formed, wherein the detection device is provided and configured to detect a drill breakthrough and/or a breakthrough tendency during drilling into a bone via the surgical drill (2), having at least one, in particular monoaxial, piezoelectric acceleration sensor (22) for generating piezoelectric acceleration signals and an evaluation unit which is provided to identify signals or signal variations characteristic of the breakthrough and/or breakthrough tendency, **characterized in that**
the piezoelectric acceleration sensor (22) detects structure-borne sound signals which are transmitted as analog signals to the evaluation unit.

2. Surgical drill (2) according to claim 1, wherein the evaluation unit includes at least one amplifier, in particular with a gain greater than/ equal to ten, and/or a filter, in particular an n-th order Butterworth filter or a bandpass filter.

3. Surgical drill (2) according to claim 2, wherein the evaluation unit includes an analog-digital converter which samples the analog signals with a sampling rate of at least 20000 Hz, in particular with a sampling rate of approximately 25600 Hz.

4. Surgical drill (2) according to claim 2 or 3, wherein the evaluation unit, preferably with a microcontroller, converts the signals into a spectrogram (40), preferably into a Mel spectrogram, via a Fourier transform, in particular a short-time Fourier transform.

5. Surgical drill (2) according to claim 4, wherein the evaluation unit evaluates the spectrogram (40) with an AI-trained network, preferably via a 'bounding box principle', and identifies the drill breakthrough or the drill breakthrough tendency when the spectrogram (40) lights up.

6. Surgical drill (2) according to claim 5, wherein the AI-trained network is a cloud network which is supplied with data by compatible detection devices.

7. Surgical drill (2) according to one of claims 1 to 6, wherein the piezoelectric acceleration sensor (22) has its highest sensitivity in a measurement frequency range up to 10000 Hz.

8. Surgical drill (2) according to one of claims 1 to 7, wherein the piezoelectric acceleration sensor (22) is coupled to a drive (16) of the surgical drill (2) by direct metallic connection.

9. Surgical drill (2) according to one of claims 1 to 8, wherein an amplifier and/or a filter of the evaluation unit is/are formed on a circuit board 24, which is/are formed, preferably hermetically sealed, in/on the surgical drill (2) in the direct vicinity of the piezoelectric acceleration sensor (22).

10. Surgical drill (2) according to one of claims 1 to 9, wherein an evaluation unit is formed completely or partially in a base unit (6) provided externally to the surgical drill.

11. Detection device which is provided and configured to detect a drill breakthrough and/or a breakthrough tendency during drilling into a bone via a surgical drill (2), having at least one, in particular monoaxial, piezoelectric acceleration sensor (22) for generating piezoelectric acceleration signals and having an evaluation unit which is provided to identify signals or signal variations characteristic of the breakthrough and/or breakthrough tendency, **characterized in that** the piezoelectric acceleration sensor (22) detects structure-borne sound signals which are transmitted as analog signals to the evaluation unit.

## Revendications

1. Perceuse (2) chirurgicale, dans ou au niveau de laquelle un appareil de détection est configuré, dans laquelle l'appareil de détection est prévu et configuré afin de détecter un perçage de foret et/ou une tendance au perçage lors du forage dans un os au moyen de la perceuse (2) chirurgicale, avec au moins un capteur d'accélération (22) piézoélectrique en particulier à un axe pour la génération de signaux d'accélération piézoélectriques et une unité d'évaluation qui est prévue afin d'identifier des signaux caractéristiques de perçage et/ou de la tendance au perçage ou des fluctuations de signal, **caractérisée en ce que** le capteur d'accélération (22) piézoélectrique détecte des signaux de structure qui sont transmis en tant que signaux analogiques à l'unité d'évaluation.

2. Perceuse (2) chirurgicale selon la revendication 1, dans laquelle
l'unité d'évaluation contient au moins un amplificateur, en particulier avec une amplification supérieure/égale à dix et/ou un filtre, en particulier un filtre Butterworth du nième ordre ou un filtre passe-bas.

3. Perceuse (2) chirurgicale selon la revendication 2, dans laquelle
l'unité d'évaluation contient un convertisseur analogique-numérique qui balaye les signaux analogiques avec un taux de balayage d'au moins 20 000 Hz, en particulier avec un taux de balayage d'environ 25 600 Hz.

4. Perceuse (2) chirurgicale selon la revendication 2 ou 3, dans laquelle
l'unité d'évaluation convertit les signaux, de préférence avec un microcontrôleur, au moyen d'une transformation de Fourier, en particulier une transmission de Fourier à court terme, en un spectrogramme (40), de préférence en un spectrogramme mel.

5. Perceuse (2) chirurgicale selon la revendication 4, dans laquelle
l'unité d'évaluation évalue le spectrogramme (40) avec un réseau formé par AI, de préférence au moyen d'un « principe de boîte de délimitation » et identifie le perçage de foret ou la tendance au perçage de foret lorsque le spectrogramme (40) est allumé.

6. Perceuse (2) chirurgicale selon la revendication 5, dans laquelle le réseau formé par AI est un réseau cloud qui est alimenté en données par des dispositifs de détection compatibles.

7. Perceuse (2) chirurgicale selon l'une quelconque des revendications 1 à 6, dans laquelle le capteur d'accélération (22) piézoélectrique possède sa plus haute sensibilité dans une plage de fréquence de mesure allant jusqu'à 10 000 Hz.

8. Perceuse (2) chirurgicale selon l'une quelconque des revendications 1 à 7, dans laquelle le capteur d'accélération (22) piézoélectrique est couplé à un entraînement (16) de la perceuse (2) chirurgicale par une liaison métallique directe.

9. Perceuse (2) chirurgicale selon l'une quelconque des revendications 1 à 8, dans laquelle un amplificateur et/ou un filtre de l'unité d'évaluation est/sont configuré(s) sur une platine (24) qui est configurée à proximité directe du capteur d'accélération (22) piézoélectrique, de préférence de manière hermétiquement étanche dans/au niveau de la perceuse (2) chirurgicale.

10. Perceuse (2) chirurgicale selon l'une quelconque des revendications 1 à 9, dans laquelle une unité d'évaluation est configurée complètement ou partiellement dans une unité de base (6) prévue à l'extérieur de la perceuse chirurgicale.

11. Appareil de détection qui est prévu et configuré afin de détecter un perçage de foret et/ou une tendance au perçage lors du forage dans un os au moyen d'une perceuse (2) chirurgicale, avec au moins un capteur d'accélération (22) piézoélectrique, en particulier à un axe pour la génération de signaux d'accélération piézoélectriques et une unité d'évaluation qui est prévue afin d'identifier des signaux caractéristiques de perçage et/ou de tendance au perçage ou des fluctuations de signal, **caractérisé en ce que** le capteur d'accélération (22) piézoélectrique détecte des signaux de structure qui sont transmis en tant que signaux analogiques à l'unité d'évaluation.
